# EUROPEAN PATENT SPECIFICATION

(11) **EP 0 796 998 B1**
(45) Date of publication and mention of the grant of the patent: **21.05.2003**
(21) Application number: 97104549.7
(22) Date of filing: 17.03.1997
(51) Int. Cl.: A61M 1/16, F04B 43/12

(54) **Dialysis unit**
Dialyseeinheit
Unite de Dialyse

(30) Priority: 18.03.1996 IT BO960150
(43) Date of publication of application: 24.09.1997
(73) Proprietor: BELLCO S.p.A., 20121 Milano (IT)
(72) Inventor: Cianciavicchia, Domenico, 64040 Cavuccio (IT)
(74) Representative: Cerbaro, Elena, Dr.

(56) References cited:
- EP-A- 0 403 401
- EP-A- 0 678 301
- US-A- 3 878 095
- US-A- 4 137 168
- US-A- 4 769 132
- US-A- 4 889 635
- US-A- 5 052 900

## Description

The present invention relates to a dialysis unit.

As is known, one of the main problems of current dialysis units is in determining the weight loss of the patient during treatment. More specifically, currently used units feature a number of peristaltic pumps for pumping fluids along the various conduits of the unit, and sophisticated, high-cost devices for determining the weight loss of the patient by measuring fluid flow along the conduits. As fluid flow is measured using invasive members, i.e. members coming into contact with the fluid circulating along the conduits, the members must be variously sterilized between one treatment and the next. If the peristaltic pumps were accurate enough, however, the flow measuring members could be dispensed with, and the weight loss of the patient determined by simply determining the functional characteristics of the pumps and so working out the resulting fluid flow. Unfortunately, however, the accuracy of peristaltic pumps falls far short of that required for dialysis treatment, which, to prevent cardiac imbalance, requires close monitoring of the weight loss of the patient throughout the treatment.

US-A-4137168 discloses a device for dialysation of blood comprising a dialyzer, a first conduit for supplying a dialysis fluid to the dialyzer, a first pump for controlling the flow of the dialysis fluid along the first conduit, a vessel for receiving the dialysis fluid from the dialyzer, a second conduit for supplying the dialysis fluid to the vessel, and a second pump for controlling the flow of the dialysis fluid along the second conduit. The device further comprises a third bypassing conduit connecting the first conduit to the second conduit, intercepting means arranged on the first, second and third conduits for selectively bypassing the dialyzer, and an assembly installed along the second conduit downstream from the third conduit.

US-A-3878095 discloses a device for dialysation of blood comprising a dialyzer, a first conduit for supplying a dialysis fluid to the dialyzer, a first pump for controlling the flow of the dialysis fluid along the first conduit, a second conduit for supplying the dialysis fluid from the dialyzer, a third bypassing conduit connecting the first conduit to the second conduit, intercepting means arranged on the first, second and third conduits for selectively bypassing the dialyzer, and an assembly installed along the second conduit downstream from the third conduit.

However, the devices disclosed in US-A-4137168 and in US-A-3878095 do not deal with the problem of determining the weight loss of the patient during treatment.

US-A-4769132 discloses a device for dialysation of blood comprising a dialyzer, a first conduit for supplying a dialysis fluid to the dialyzer, a first pump for controlling the flow of the dialysis fluid along the first conduit, a vessel for receiving the dialysis fluid from the dialyzer, a second conduit for supplying the dialysis fluid to the vessel, and a second pump for controlling the flow of the dialysis fluid along the second conduit. The vessel is positioned on a load cell which activates the second pump according to a measured change in weight of the vessel and, thus, of the patient during the treatment of dialysis.

However, the device disclosed in US-A-4769132 has a relatively low precision in calculating a weight loss of a patient undergoing the treatment of dialysis.

It is an object of the present invention to provide a dialysis unit designed to overcome the aforementioned drawbacks, i.e. which provides for highly accurate control of the weight loss of the patient.

According to the present invention, there is provided a dialysis unit as recited in Claim 1.

A preferred, non-limiting embodiment of the present invention will be described by way of example with reference to the accompanying drawings, in which:
Figure 1 shows a block diagram of a dialysis unit in accordance with the teachings of the present invention;
Figure 2 shows a member forming part of the Figure 1 unit;
Figures 3 and 4 show a different embodiment of the Figure 2 member in two different operating modes.

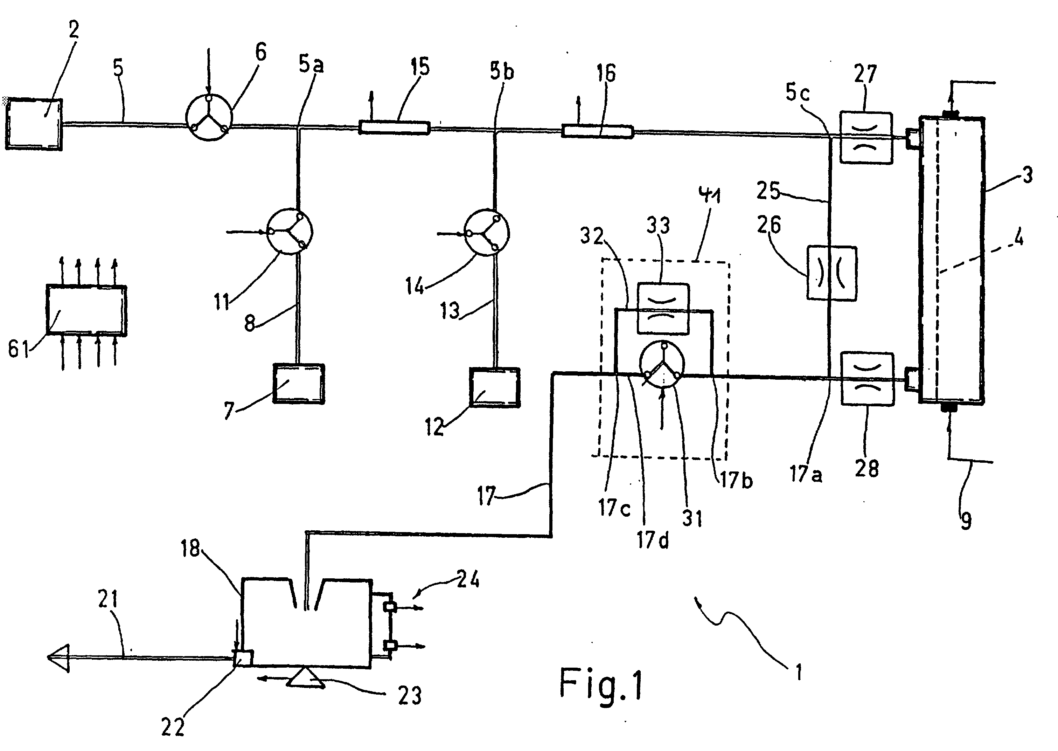

Number 1 in Figure 1 indicates a dialysis unit, which is substantially defined by three parts: a first part in which the dialysis solution is prepared; a second part in which the dialysis solution is used; and a third part for draining the fluid from the second part and controlling the weight loss of the patient.

The second part of unit 1 comprises a known dialyzer 3 in which a membrane 4 defines two channels, a first for extracorporeal blood flowing along a conduit 9, and a second for the dialysis fluid.

The first part of unit 1 comprises:
a water source 2;
a conduit 5 having a first end connected to source 2, and a second end connected to the second channel of dialyzer 3 and from which the dialysis fluid is released;
a known peristaltic pump 6 along conduit 5;
a salt concentrate and water solution source 7;
a conduit 8 hydraulically connecting source 7 to a mixing point 5a defined along conduit 5, downstream from pump 6;
a known peristaltic pump 11 along conduit 8;
a salt concentrate and water solution source 12;
a conduit 13 hydraulically connecting source 12 to a mixing point 5b defined along conduit 5, downstream from point 5a;
a known peristaltic pump 14 along conduit 13;
a preferably conductometric sensor 15 for determining the composition of the fluid fed along conduit 5 between mixing points 5a and 5b; and
a preferably conductometric sensor 16 for determining the composition of the fluid fed along conduit 5 downstream from mixing point 5b.

The third part of unit 1 comprises:
a conduit 17 connected at a first end to the second channel of dialyzer 3;
a vessel 18 housing the second end of conduit 17;
a conduit 21 extending from an outlet formed in vessel 18 to a drain (not shown);
a solenoid valve 22 fitted to conduit 21 at said outlet of vessel 18;
electronic weighing means 23 for determining the weight of vessel 18 and its contents;
electronic means 24 for determining the level of the contents of vessel 18;
a conduit 25 hydraulically connecting a point 5c, defined downstream from sensor 16 along conduit 5, to a point 17a along conduit 17;
fluid intercepting means 26 along conduit 25;
fluid intercepting means 27 along conduit 5, downstream from point 5c;
fluid intercepting means 28 along conduit 17, upstream from point 17a;
a peristaltic pump 31 along conduit 17, downstream from point 17a, and more specifically between two points 17b and 17c between which is defined a branch 17d of conduit 17;
a conduit 32 parallel to branch 17d and therefore hydraulically connecting points 17b and 17c of conduit 17; and
fluid intercepting means 33 along conduit 32.

Intercepting means 26, 27, 28 and 33 may be controlled manually and comprise, for example, straightforward grippers, or may be controlled electrically and comprise, for example, solenoid valves. Pump 31, branch 17d, conduit 32 and intercepting means 33 define an assembly 41, which provides for two different operating modes. More specifically, assembly 41 may permit flow along conduit 32 only, by cutting off branch 17d by means of pump 31 (as explained in detail later on), or may permit flow along branch 17d only by operating pump 31 and cutting off conduit 32.

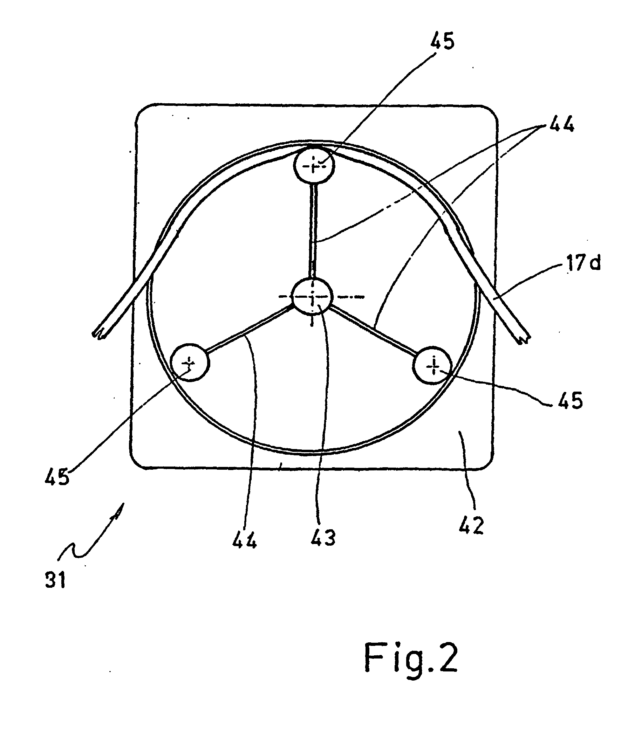

Pump 31 of unit 1 in Figure 1 is a typical peristaltic pump and, as shown schematically in Figure 2, comprises an elongated hollow cylindrical body 42; a rotary shaft 43 rotated by an electric motor (not shown); three arms 44 extending radially from shaft 43 and spaced 120° apart; and idle wheels 45, each fitted to the free end of a respective arm 44. Shaft 43 is coaxial with body 42, which houses arms 44; the length of arms 44 and the diameter of wheels 45 are such that wheels 45 substantially contact the inner surface of body 42; and a portion of the inner surface of body 42 extending about an arc of over 120° houses a portion of conduit 17, so that, when pump 31 is turned off, at least one of wheels 45 presses on said conduit portion, cutting off all flow along conduit 17. As shaft 43 rotates, wheels 45 naturally exert pressure successively along the whole of said portion of conduit 17, thus producing the known pumping effect.

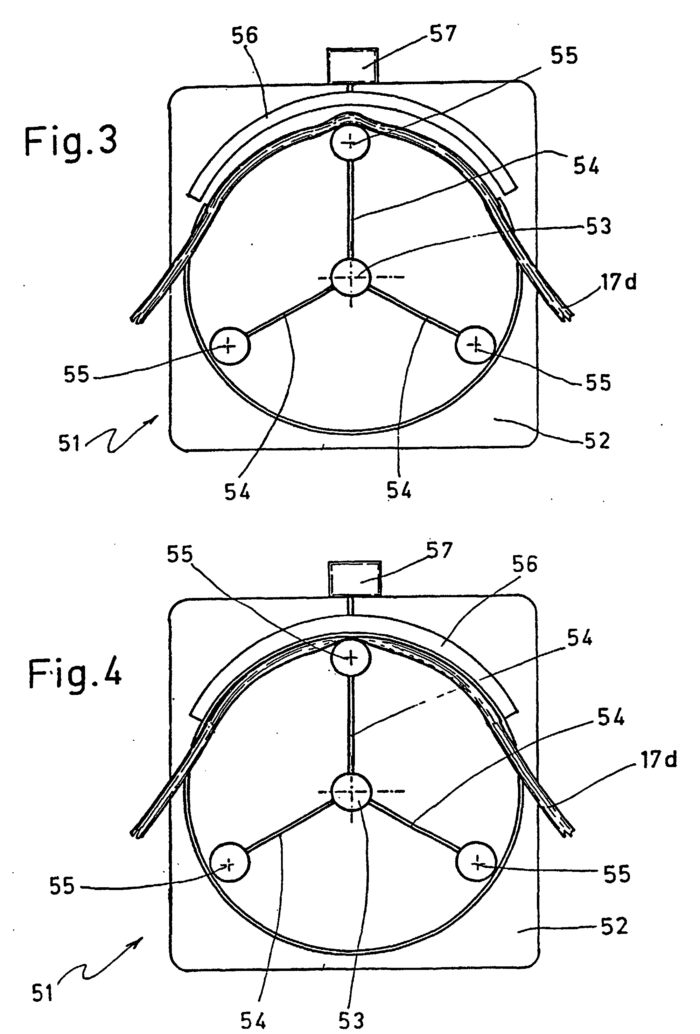

Number 51 in Figures 3 and 4 indicates a known pump, which may be fitted between points 17b and 17c of conduit 17 in place of assembly 41, and which, like assembly 41, provides for two different operating modes. More specifically, pump 51 is designed to permit free flow along branch 17d when disabled, and controlled flow along branch 17d when operated as a normal peristaltic pump. Pump 51 comprises an elongated body 52 formed from a cylindrical body (similar to body 42) from which is removed an axial portion extending in section about an angle of over 120°; a rotary shaft 53 rotated by an electric motor knot shown); three arms 54 extending radially from shaft 53 and spaced 120° apart; and idle wheels 55, each fitted to the free end of a respective arm 54. Shaft 53 is coaxial with body 52, which houses arms 54; and the length of arms 54 and the diameter of wheels 55 are such that wheels 55 substantially contact the inner surface of body 52. Pump 51 also comprises an elongated plate 56 extending in section about an arc of a radius equal to the inside radius of body 52. That is, plate 56 is so formed as to substantially define said portion removed from the cylindrical body from which body 52 is formed, and is movable, via actuating means 57, between a first position in which the inner surface of plate 56 is a continuation of the inner surface of body 52, and a second position in which the distance between the inner surface of plate 56 and the longitudinal axis of body 52 is greater than the inside radius of body 52. In said first position, the inner surface of plate 56 houses a portion of conduit 17, so that, when pump 51 is turned off, at least one of wheels 55 presses on said conduit portion, cutting off all flow along conduit 17. As shaft 53 rotates, wheels 55 naturally exert pressure successively along the whole of said portion of conduit 17, thus producing the known pumping effect. When plate 56 is set to said second position, free flow is permitted along conduit 17.

With reference to Figure 1, unit 1 also comprises an electronic central control unit 61 for controlling operation of unit 1, and more specifically all the peristaltic pumps, solenoid valve 22, and the intercepting means if these are electrically operated, and which is connected to sensors 15, 16 and means 23, 24.

The functioning of the dialysis unit substantially comprises two main operating cycles, the first of which provides for self-calibrating the flow of dialysis fluid along conduit 5 downstream from point 5b, and the second for determining and controlling the quantity of substances extracted from the blood at dialyzer 3, and hence the weight loss of the patient.

The first cycle comprises:
hydraulically isolating dialyzer 3, i.e. disabling hydraulic communication between dialyzer 3 and the rest of unit 1 via intercepting means 27 and 28;
allowing the dialysis solution to flow along conduit 25 via intercepting means 26;
enabling free flow of the dialysis solution between points 17b and 17c with the pump disabled; that is, enabling free flow along conduit 32 and arresting pump 31 to close branch 17d, when using assembly 41, and maintaining plate 56 in said second position, when using pump 51;
activating solenoid valve 22 to cut off flow along conduit 21;
enabling pumps 6, 11 and 14;
determining the composition of the dialysis solution in known manner by means of sensors 15 and 16;
determining, via weighing means 23, the flow rate of dialysis solution along conduit 5, downstream from point 5b, which is equal to the flow of dialysis solution along conduit 17, downstream from point 17a; and
adjusting control of pumps 6, 11 and 14 to achieve the predetermined flow and composition of dialysis solution, and memorizing the control parameters of pumps 6, 11 and 14 by which said predetermined flow and composition are determined.

The second cycle comprises:
cutting off all flow to conduit 25;
enabling hydraulic communication to and from dialyzer 3, i.e. enabling flow along conduit 5, downstream from point 5c, and along conduit 17, upstream from point 17a;
enabling operation of pumps 6, 11 and 14 according to the memorized control parameters;
peristaltic pump controlling flow between points 17b and 17c; that is, closing conduit 32 and enabling pump 31, when using assembly 41, and moving plate 56 into said first position and enabling pump 51, when using pump 51;
adjusting the control parameters of pump 31 or 51 until means 23 determine a flow rate between points 17b and 17c equal to the predetermined flow rate calibrated in the first cycle; and
adjusting the control parameters of pump 31 or 51 (increasing the rotation speed of shaft 43 or 53) until the flow rate along conduit 17 is greater than the constant flow rate along conduit 5, the difference in flow rate being detected by means 23 and being equal to a predetermined weight loss of the patient.

Means 24 may be used in place of or in conjunction with means 23, so that flow into vessel 18 may be determined either by weighing (means 23) and/or by determining the level of the fluid fed in time into vessel 18. Means 24 may also be used to determine when a predetermined threshold level is exceeded, in which case, the fluid in vessel 18 is drained off by solenoid valve 22. Self-calibration of the pumps and control of the weight loss of the patient as described above may be performed repeatedly during treatment to control the weight loss of the patient more accurately and possibly also to adjust at various times during treatment both the dialysis solution flow and composition parameters and the desired weight loss.

The advantages of the present invention will be clear from the foregoing description.

In particular, it provides for a unit enabling highly accurate control of the weight loss of the patient, by virtue of control not requiring detection of the functional characteristics of the peristaltic pumps to determine the flow rates involved. Finally, the unit itself is straightforward in design, and requires no invasive flow detecting devices, and hence, no sterilization between one treatment and the next.

## Claims

1. A dialysis unit comprising:
a dialyzer (3) in which a membrane (4) defines two channels, a first for extracorporeal blood, and a second for a dialysis fluid;
a first conduit (5) along which said dialysis fluid flows to said second channel of said dialyzer (3);
first pumping means (6, 11, 12) for controlling the flow of said dialysis fluid along said first conduit (5);
a vessel (18) for receiving the fluid from said second channel of said dialyzer (3);
a second conduit (17) along which said fluid from said dialyzer (3) flows to said vessel (18); and
sensor means (15, 16) for determining the composition of said dialysis fluid flowing along said first conduit (5);
a third conduit (25) for hydraulically connecting a first point (5c) of said first conduit (5) upstream from said dialyzer (3) to a second point (17a) of said second conduit (17) downstream from said dialyzer (3);
first intercepting means (26, 27, 28) installed along said third conduit (25), along said first conduit (5) downstream from said first point (5c), and along said second conduit (17) upstream from said second point (17a); and
an assembly (41, 51) installed along said second conduit (17), between a third point (17b) downstream from said second point (17a), and a successive fourth point (17c);
**characterized by** the fact that:
the assembly (41, 51) permits flow between said third (17b) and fourth (17c) points freely at one stage, and via second pumping means (31, 51) at another stage;
means (23, 24) being fitted to said vessel (18) to determine the flow rate of said fluid fed from said second conduit (17) into said vessel (18).

2. A unit as claimed in Claim 1, **characterized in that**, between said third (17b) and fourth (17c) points, said assembly (41) comprises a first branch (17d) along which are installed said second pumping means defined by a first peristaltic pump (31), which, when disabled, cuts off flow along said first branch (17d), and, when enabled, generates a pumping effect along said first branch (17d); and a second branch (32) parallel to the first, and along which are installed second intercepting means (33).

3. A unit as claimed in Claim 1, **characterized in that** said assembly comprises a peristaltic pump (51), which, when disabled, permits free flow along said second conduit (17), and, when enabled, generates a pumping effect along said second conduit (17).

4. A unit as claimed in Claim 2 or 3, **characterized by** comprising a first source (2) of water from which said first conduit (5) originates; a second source (7) of a salt concentrate and water solution from which originates a fourth conduit (8) connected to a fifth point (5a) of said first conduit (5); and a third source (12) of a salt concentrate and water solution from which originates a fifth conduit (13) connected to a sixth point (5b) of said first conduit (5); said first pumping means comprising a second peristaltic pump (6) installed along said first conduit (5) upstream from said fifth (5a) and sixth (5b) points, a third peristaltic pump (11) installed along said fourth conduit (8), and a fourth peristaltic pump (14) installed along said fifth conduit (13).

5. A unit as claimed in any one of the foregoing Claims, **characterized in that** said means for determining the flow rate of said fluid fed into said vessel (18) comprise a weighing device (23) for weighing said vessel (18).

6. A unit as claimed in any one of the foregoing Claims, **characterized in that** said means for determining the flow rate of said fluid fed into said vessel (18) comprise a detecting device (24) for detecting the level of said fluid in said vessel (18).

7. A unit as claimed in any one of the foregoing Claims, **characterized by** comprising an electronic central control unit (61) for controlling said first (6, 11, 14) and second (31, 51) pumping means, and to which are connected said sensor means (15, 16) and said means (23, 24) for determining the flow rate of the fluid fed into said vessel (18).

## Patentansprüche

1. Eine Dialyseeinheit, welche umfaßt:
einen Dialysator (3), in welchem eine Membran (4) zwei Kanäle definiert, einen ersten für extrakorporales Blut und einen zweiten für ein Dialysefluid;
eine erste Leitung (5), entlang welcher das Dialysefluid zu dem zweiten Kanal des Dialysators (3) fließt;
ein erstes Pumpmittel (6, 11, 12) zum Kontrollieren des Flusses des Dialysefluids entlang der ersten Leitung (5);
ein Gefäß (18) zum Empfangen des Fluids von dem zweiten Kanal des Dialysators (3);
eine zweite Leitung (17), entlang welcher das Fluid von dem Dialysator (3) zu dem Gefäß (18) fließt; und
ein Sensoxmittel (15, 16) zum Bestimmen der Zusammensetzung des Dialysefluids, das entlang der ersten Leitung (5) fließt;
eine dritte Leitung (25) zum hydraulischen verbinden eines ersten Punktes (5c) der ersten Leitung (5) stromaufwärts von dem Dialysator (3) zu einem zweiten Punkt (17a) der zweiten Leitung (17) flußabwärts von dem Dialysator (3);
ein erstes Unterbrechungsmittel (intercepting means) (26, 27, 28), installiert entlang der dritten Leitung (25), entlang der ersten Leitung (5) flußabwärts von dem ersten Punkt (5c) und entlang der zweiten Leitung (17) flußaufwärts von dem zweiten Punkt (17a); und
einen Aufbau (assembly) (41, 51), installiert entlang der zweiten Leitung (17) zwischen einem dritten Punkt (17b) stromabwärts von dem zweiten Punkt (17a) und einem sich anschließenden vierten Punkt (17c) ;
durch die Tatsache gekennzeichnet, daß:
der Aufbau (41, 51) in einem Stadium einen freien Fluß zwischen dem dritten (17b) und vierten (17c) Punkt erlaubt und in einem anderen Stadium einen Fluß über zweite Pumpmittel (31, 51) erlaubt;
Mittel (23, 24) an das Gefäß (18) angepaßt sind, um die Flußrate des Fluids, das aus der zweiten Leitung (17) in das Gefäß (18) eingeleitet wird, zu bestimmen.

2. Eine Einheit nach Anspruch 1, **dadurch gekennzeichnet, daß** zwischen dem dritten (17b) und vierten (17c) Punkt der Aufbau (41) einen ersten Zweig (17d), entlang dem die zweiten Pumpmittel, die durch eine erste peristaltische Pumpe (31) deliniert werden, welche, wenn sie abgeschaltet ist, den Fluß entlang des ersten Zweiges (17d) abschneidet und, wenn sie angeschaltet ist, einen Pumpeffekt entlang des ersten Zweiges (17d) produziert, installiert sind; und einen zweiten Zweig (32) parallel zu dem ersten, und entlang dem zweite Unterbrechungsmittel (33) installiert sind, umfaßt.

3. Eine Einheit nach Anspruch 1, **dadurch gekennzeichnet, daß** der Aufbau eine peristaltische Pumpe (51) umfaßt, welche, wenn sie abgeschaltet ist, einen freien Fluß entlang der zweiten Leitung (17) erlaubt und, wenn sie angeschaltet ist, einen Pumpeffekt entlang der zweiten Leitung (17) generiert.

4. Eine Einheit nach Anspruch 2 oder 3, **dadurch gekennzeichnet, daß** sie umfaßt:
eine erste Wasserquelle (2), an welcher die erste Leitung (5) beginnt; eine zweite Quelle (7) einer konzentrierten Salz- und Wasserlösung, an welcher eine vierte Leitung (8) beginnt, die mit einem fünften Punkt (5a) der ersten Leitung (5) verbunden ist; und eine dritte Quelle (12) einer konzentrierten Salz- und Wasserlösung, an welcher eine fünfte Leitung (13) beginnt, die mit einem sechsten Punkt (5b) der ersten Leitung (5) verbunden ist; wobei das erste Pumpmittel eine zweite peristaltische Pumpe (6) umfaßt, die entlang der ersten Leitung (5) stromaufwärts von dem fünften (5a) und sechsten (5b) Punkt installiert ist, eine dritte peristaltische Pumpe (11), die entlang der vierten Leitung (8) installiert ist, und eine vierte peristaltische Pumpe (14), die entlang der fünften Leitung (13) installiert ist.

5. Eine Einheit nach einem der vorangehenden Ansprüche, **dadurch gekennzeichnet, daß** die Mittel zum Bestimmen der Flußrate des Fluids, das in das Gefäß (18) eingeleitet wird, ein Wichtungsmittel (23) zum Wiegen des Gefäßes (18) umfassen.

6. Eine Einheit nach einem der vorangehenden Ansprüche, **dadurch gekennzeichnet, daß** die Mittel zum Bestimmen der Flußrate des Fluids, das in das Gefäß (18) eingeleitet wird, ein Detektionsmittel (24) zum Detektieren des Spiegels des Fluids in dem Gefäß (18) umfassen.

7. Eine Einheit nach einem der vorangehenden Ansprüche, **dadurch gekennzeichnet, daß** sie eine elektronische zentrale Kontrolleinheit (61) zum Kontrollieren der ersten (6, 11, 14) und zweiten (31, 51) Pumpmittel umfaßt, und mit welcher die Sensormittel (15, 16) und die Mittel (23, 24) zum Bestimmen der Flußrate des Fluids, das in das Gefäß (18) eingeführt wird, verbunden sind.

## Revendications

1. Unité de dialyse comprenant :
un dialyseur (3) dans lequel une membrane (4) définit deux canaux, un premier destiné à du sang extracorporel, et un deuxième destiné à un liquide de dialyse ;
une première canalisation (5) le long de laquelle ledit liquide de dialyse s'écoule vers ledit deuxième canal dudit dialyseur (3) ;
des premiers moyens de pompage (6, 11, 12) destinés à réguler l'écoulement dudit liquide de dialyse le long de ladite première canalisation (5) ;
un récipient (18) destiné à recevoir le liquide provenant dudit deuxième canal dudit dialyseur (3) ;
une deuxième canalisation (17) le long de laquelle ledit liquide provenant dudit dialyseur (3) s'écoule vers ledit canal (18) ; et
des moyens de détecteur (15, 16) destinés à déterminer la composition dudit liquide de dialyse s'écoulant le long de ladite première canalisation (5) ;
une troisième canalisation (25) destinée à relier hydrauliquement un premier point (5c) de ladite première canalisation (5) en amont dudit dialyseur (3) à un deuxième point (17a) de ladite deuxième canalisation (17) en aval dudit dialyseur (3) ;
des premiers moyens d'interruption d'écoulement (26, 27, 28) installés le long de ladite troisième canalisation (25), le long de ladite première canalisation (5) en aval dudit premier point (5c), et le long de ladite deuxième canalisation (17) en amont dudit deuxième point (17a) ; et
un assemblage (41, 51) installé le long de ladite deuxième canalisation (17), entre un troisième point (17b) en aval dudit deuxième point (17a), et un quatrième point (17c) successif ;
**caractérisée par le fait que** :
l'assemblage (41, 51) permet un écoulement, entre lesdits troisième (17b) et quatrième (17c) points, libre dans une première phase, et par l'intermédiaire des deuxièmes moyens de pompage (31, 51) dans une deuxième phase ;
des moyens (23, 24) étant fixés audit récipient (18) pour déterminer le débit dudit liquide introduit à partir de ladite deuxième canalisation (17) dans ledit récipient (18).

2. Unité selon la revendication 1, **caractérisée en ce que**, entre lesdits troisième (17b) et quatrième (17c) points, ledit assemblage (41) comprend une première branche (17d) le long de laquelle lesdits deuxièmes moyens de pompage sont installés, définis par une première pompe péristaltique (31), qui, quand elle est désactivée, coupe l'écoulement le long de ladite première branche (17d), et, quand elle est activée, engendre un effet de pompage le long de ladite première branche (17d) ; et une seconde branche (32) parallèle à la première, et le long de laquelle les deuxièmes moyens d'interruption d'écoulement (33) sont installés.

3. Unité selon la revendication 1, **caractérisée en ce que** ledit assemblage comprend une pompe péristaltique (51), qui, quand elle est désactivée, permet un écoulement libre le long de ladite deuxième canalisation (17), et, quand elle est activée, engendre un effet de pompage le long de ladite deuxième canalisation (17).

4. Unité selon la revendication 2 ou 3, **caractérisée en ce qu'**elle comprend une première source (2) d'eau d'où part la première canalisation (5) ; une deuxième source (7) d'un concentré de sel et d'une solution aqueuse d'où part une quatrième canalisation (8) reliée à un cinquième point (5a) de ladite première canalisation (5) ; et une troisième source (12) d'un concentré de sel et d'une solution aqueuse d'où part une cinquième canalisation (13) reliée à un sixième point (5b) de ladite première canalisation (5) ; lesdits premiers moyens de pompage comprenant une deuxième pompe péristaltique (6) installée le long de ladite première canalisation (5) en amont desdits cinquième (5a) et sixième (5b) points, une troisième pompe péristaltique (11) installée le long de ladite quatrième canalisation (8), et une quatrième pompe péristaltique (14) installée le long de ladite cinquième canalisation (13).

5. Unité selon l'une quelconque des revendications précédentes, **caractérisée en ce que** lesdits moyens destinés à déterminer le débit dudit liquide introduit dans ledit récipient (18) comprennent un dispositif de pesée (23) destiné à peser ledit récipient (18).

6. Unité selon l'une quelconque des revendications précédentes, **caractérisée en ce que** lesdits moyens destinés à déterminer le débit dudit liquide introduit dans ledit récipient (18) comprennent un dispositif de détection (24) destiné à détecter le niveau dudit liquide dans ledit récipient (18).

7. Unité selon l'une quelconque des revendications précédentes, **caractérisée en ce qu'**elle comprend une unité de commandé centrale électronique (61) destinée à commander lesdits premiers (6, 11, 14) et seconds (31, 51) moyens de pompage, et à laquelle lesdits moyens de détecteur (15, 16) et lesdits moyens (23, 24) destinés à déterminer le débit du liquide introduit dans ledit récipient (18), sont connectés.
